# EUROPÄISCHE PATENTSCHRIFT

(11) **EP 1 097 122 B1**
(45) Veröffentlichungstag und Bekanntmachung des Hinweises auf die Patenterteilung: **22.09.2004**
(21) Anmeldenummer: 99938227.8
(22) Anmeldetag: 13.07.1999
(51) Int. Cl.: C07C 209/16

(54) **VERFAHREN ZUR HERSTELLUNG VON AMINEN**
METHOD FOR PRODUCING AMINES
PROCEDE DE PRODUCTION D'AMINES

(30) Priorität: 21.07.1998 EP 98113540
(43) Veröffentlichungstag der Anmeldung: 09.05.2001
(73) Patentinhaber: LONZA A.G., CH-4002 Basel (CH)
(72) Erfinder: BAIKER, Alfons, CH-8152 Opfikon (CH); FISCHER, Achim, CH-8057 Zürich (CH); MALLAT, Tamas, CH-8057 Zürich (CH); WERBITZKY, Oleg, CH-3930 Visp (CH)
(86) Internationale Anmeldenummer: PCT/EP1999/004901
(87) Internationale Veröffentlichungsnummer: WO 2000/005190

(56) Entgegenhaltungen:
- EP-A- 0 839 796
- US-A- 3 331 877
- US-A- 4 014 933
- US-A- 4 123 462
- US-A- 5 099 070
- US-A- 5 288 911

## Beschreibung

Die vorliegende Erfindung betrifft ein Verfahren zur Herstellung von primären aliphatischen Polyaminen aus den entsprechenden Aminoalkoholen und/oder Polyalkoholen. Die erfindungsgemäss herstellbaren Polyamine besitzen die allgemeine Formel 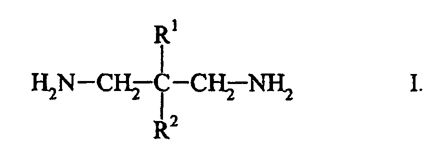 Hierin bedeuten R¹ und R² unabhängig voneinander Wasserstoff, Methyl, Ethyl oder Aminomethyl.

Es ist bekannt, dass einige primäre Diamine aus den entsprechenden Alkoholen oder Aminoalkoholen hergestellt werden können. Das technisch wichtigste Verfahren dieser Art ist die Herstellung von 1,2-Ethandiamin (Ethylendiamin) aus Ethanolamin. Es gibt allerdings nur wenige Verfahren zur analogen Herstellung von aliphatischen Polyaminen der Formel I, deren Effizienz zudem gering ist. So werden stattdessen aufwendig herzustellende Ausgangsverbindungen oder komplexe Herstellungsverfahren gewählt und gewöhnlich nur geringe Ausbeuten erhalten.
In US-A-4 123 462 wird eine Methode zur Herstellung von Aminen unter Verwendung eines Nickel-Rhenium-Katalysators offenbart. In den angeführten Beispielen wird die Aminierung von 1,3-Propandiol mit einem Umsatz von 45% angegeben. Angaben über die Produkteausbeuten oder -selektivitäten werden nicht genannt.
In US-A-4 158 017 wird als Ausgangsverbindung Hydroxypivalaldehyd eingesetzt, um das entsprechende Diamin (2,2-Dimethyl-1,3-propandiamin, "Neopentyldiamin") zu erhalten. Hydroxypivalaldehyd ist eine aufwendig herzustellende Ausgangsverbindung, deren Aminierung mehrere Reaktionsstufen benötigt. Deswegen ist dieses Verfahren wenig wirtschaftlich.

Gemäss US-A-5 099 070 ist es für die Herstellung von Neopentyldiamin durch Umsetzung von Neopentanolamin (3-Amino-2,2-dimethyl-1-propanol) mit Ammoniak in Gegenwart von Wasserstoff an einem Nickelkatalysator wesentlich, dass das Ausgangsmaterial frei von Neopentylglycol ist, um mögliche Nebenreaktionen des Diols wie die Hydrogenolyse zu vermeiden.
In US-A-5 288 911 wird ein Katalysator aus Cobalt und Eisen verwendet, um aus α,ω-Alkandiolen bevorzugt α,ω-Aminoalkohole zu produzieren. Aus den offenbarten Beispielen ist ersichtlich, dass das Diamin nur als Nebenprodukt erhalten wird. Der aufgezeigte Prozess ist daher nicht zur technischen Herstellung des Diamins geeignet.
Im Stand der Technik ist kein Verfahren offenbart, durch das primäre aliphatische Polyamine mit mehr als zwei terminalen Aminogruppen aus dem entsprechenden Alkohol der Formel I direkt erhalten werden können. Die industrielle Herstellung von Verbindungen dieser Produktklasse, z. B. 2-(Aminomethyl)-2-methyl-1,3-propandiamin ("1,1,1-Tris(aminomethyl)ethan"), 2-(Aminomethyl)-2-ethyl-1,3-propandiamin ("1,1,1-Tris(aminomethyl)-propan"), oder 2,2-Bis(aminomethyl)-1,3-propandiamin ("Pentaerythrityltetramin") aus den entsprechenden leicht zugänglichen Polyalkoholen ist jedoch besonders attraktiv. Eine bekannte mehrstufige Synthese (E. B. Fleischer et al., *J. Org. Chem.* **1971**, 36, 3042) benötigt das Azid als Zwischenprodukt. Azide neigen zur explosionsartigen Zersetzung, was sie für technische Verfahren wenig geeignet macht.

Aufgabe der vorliegenden Erfindung war daher, ein für die Durchführung in technischem Massstab geeignetes Verfahren bereitzustellen, das in einer Stufe primäre aliphatische Polyamine aus den entsprechenden Polyolen liefert.

Erfindungsgemäss wird diese Aufgabe durch das Verfahren nach Patentanspruch 1 gelöst.

Es wurde gefunden, dass aus primären aliphatischen Polyalkoholen der allgemeinen Formel 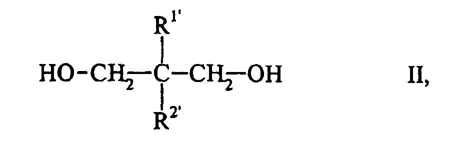 worin R^{1'} die oben für R¹ genannte Bedeutung hat oder, falls R¹ Aminomethyl ist, R^{1'} auch Hydroxymethyl bedeuten kann,
und R^{2'} die oben für R² genannte Bedeutung hat oder, falls R² Aminomethyl ist, R^{2'} auch Hydroxymethyl bedeuten kann,
durch Umsetzung mit Ammoniak in Gegenwart von Wasserstoff die entsprechenden primären Polyamine in einem einstufigen Verfahren erhalten werden können, wenn ein in seinem Metallanteil 5-95 Gewichtsprozent Nickel und/oder Cobalt enthaltender Katalysator eingesetzt wird, der zusätzlich einen gehalt von 1-60 Gewichtsprozent Fe und/oder La besitzt, und das Ammoniak sich im überkritischen Zustand, d. h. unter einem Druck von mehr als 113 bar und bei einer Temperatur von mehr als 133 °C, befindet und das Mengenverhältnis 1-300 mol Ammoniak und
0,01-20 mol Wasserstoff auf 1 Äquivalent (val) Polyalkohol II beträgt.

Vorzugsweise liegt der Druck bei 115-300 bar und die Temperatur bei 150-300 °C.

Besonders bevorzugt sind Drücke von 120-200 bar und Temperaturen von 160-250 °C.

Das Mengenverhältnis beträgt vorzugsweise 10-100 mol, besonders bevorzugt 40-90 mol, Ammoniak und 2-10 mol Wasserstoff auf 1 Äquivalent (val) Polyalkohol.

Es hat sich ausserdem gezeigt, dass der Katalysator nicht zu basisch oder zu acid sein sollte.

Die erfindungsgemäss einsetzbaren Katalysatoren können bespielsweise dadurch hergestellt werden, dass man die Hydroxide, Oxidhydrate und/oder Hydroxycarbonate der aktiven Metalle bei einem pH von 5-9 ausfällt, trocknet und bei 200-500 °C in oxidierender Atmosphäre kalziniert.

Eine gewisse Anzahl und Stärke von basischen und aciden Zentren auf der Katalysatoroberfläche sind für die Aminierung der Alkohole vorteilhaft, da die Adsorption und Aktivierung von Ammoniak und des Aminoalkohols, der als Zwischenprodukt auftritt, diese Zentren benötigen. Stark basische und stark saure Zentren auf der Katalysatoroberfläche sollten allerdings vermieden werden. Solche Zentren werden beispielsweise durch Reagenzien mit stark basischen Ionen wie z. B. Natrium, Kalium, Calcium, Barium oder stark aciden lonen wie z. B. Phosphat, Hydrogenphosphat induziert. Hierbei bezieht sich die Basizität bzw. Acidität auf die Wirkung an der Festkörperoberfläche und nicht auf die üblicherweise betrachteten Eigenschaften in Lösung. Es wurde gefunden, dass derartige Zentren die Selektivität der Umsetzung zu den gewünschten Produkten verschlechtern und basen- bzw. säurekatalysierte Nebenreaktionen wie Fragmentierung, Cyclisierung und Oligomerisierung begünstigt werden. Eine Möglichkeit, die sauren und basischen Eigenschaften des Katalysators zu beeinflussen, ist die Kontrolle des pH-Wertes bei der Fällung des Katalysators. Die Fällung sollte deshalb bei einem pH von 5-9 durchgeführt werden. Weiterhin ist es vorteilhaft, den Niederschlag sorgfältig zu waschen.

Die Fällung kann beispielsweise durch Zugabe einer Base zu einer Lösung der Acetate, Nitrate oder Halogenide der Metallkomponente(n) erfolgen. Bevorzugte Basen hierfür sind Ammoniumcarbonat bzw. -carbamat oder Ammoniak. Die Trocknung erfolgt vorzugsweise bei. Temperaturen bis 150 °C, gegebenenfalls im Vakuum.

Der Katalysator wird vorzugsweise bei 300-500 °C in oxidierender Atmosphäre kalziniert.

Für den Katalysator kann ein Träger wie Siliciumdioxid, Kieselgur, Aluminiumoxid oder Graphit verwendet werden. Zu diesem Zweck kann die Fällung in Gegenwart des Trägers durchgeführt werden.

Der Katalysator wird vor Gebrauch vorteilhaft durch reduzierende Gase wie z. B. Wasserstoff bei Temperaturen von 200 bis 400 °C aktiviert.

Das erfindungsgemässe Verfahren kann mit unverzweigten wie auch verzweigten Polyalkoholen II durchgeführt werden. Besonders bevorzugt sind 1,3-Propandiol, 2-Methyl-1,3-propandiol, 2,2-Dimethyl-1,3-propandiol, 1,1,1-Tris-(hydroxymethyl)-ethan und Pentaerythrit.

Das erfindungsgemässe Verfahren kann diskontinuierlich oder kontinuierlich durchgeführt werden, bevorzugt ist die kontinuierliche Arbeitsweise.

Die Kontaktzeit (definiert als Quotient aus Katalysatormasse [g] und zugeführtem Molenstrom [mol/s] der Reaktionspartner) bei kontinuierlicher Durchführung beträgt vorzugsweise 10 000 g·s/mol bis 100 000 g·s/mol.

Bei kontinuierlicher Arbeitsweise wird der nicht umgesetzte Polyalkohol und/oder das nicht umgesetzte Ammoniak vorzugsweise rezykliert. Hierdurch kann auch bei (nach einmaliger Passage) relativ geringem Umsatz eine befriedigende bis gute Produktausbeute erzielt werden.

Das Ammoniak kann dem Reaktionsgefäss als Gas, bevorzugt aber als Flüssigkeit zugeführt werden, der überkritische Zustand kann dann von der Gas- oder der Flüssigphase ausgehend erreicht werden. Die Verwendung von flüssigem Ammoniak hat bei festen Ausgangsmaterialien den Vorteil, dass diese schon vor dem Erreichen des überkritischen Zustands gelöst werden können und das Reaktionsgemisch somit von Beginn an als homogene Phase vorliegt. Das Reaktionsgemisch wird nach der Reaktion vorteilhaft gekühlt, z. B. durch adiabatische Expansion, das Ammoniak abgetrennt und gegebenenfalls in den Prozess rückgeführt.

Die nach dem erfindungsgemässen Verfahren herstellbaren aliphatischen primären Polyamine der Formel I können beispielsweise für die Polyurethanherstellung, insbesondere als Vernetzer und/oder Katalysatoren mit geringer Migrationsneigung, eingesetzt werden.

Die folgenden Beispiele verdeutlichen die Durchführung des erfindungsgemässen Verfahrens.

### Beispiel 1

### Herstellung eines Co/Fe-Katalysators

Die Salze Co(NO₃)₂·6 H₂O und Fe(NO₃)₃·9 H₂O wurden in einem molaren Verhältnis von 20 : 1 und einer Gesamtkonzentration von 0,36 mol/l in Wasser gelöst und bei pH 7 mit 100 g einer 20%igen wässrigen Lösung von handelsüblichem "Ammoniumcarbonat" (Gemisch aus Carbamat und Carbonat) versetzt, wobei Cobalt und Eisen als Hydroxycarbonate ausfielen. Der Niederschlag wurde abfiltriert, sorgfältig mit Wasser gewaschen, bei 100 °C getrocknet und bei 400 °C kalziniert. Vor Gebrauch wurde der so erhaltene Katalysator bei 330 °C mit Wasserstoff aktiviert.

### Beispiel 2

### Herstellung von 1,3-Propandiamin

Die Reaktion wurde in einem kontinuerlichen Reaktorsystem durchgeführt. Der Reaktor bestand aus einem Inconel®-718 Rohr mit einem Innendurchmesser von 13 mm und einer Länge von 304 mm. Er enthielt 8 g des Co/Fe-Katalysators aus dem vorstehenden Beispiel. Ein Reaktionsgemisch aus 1,3-Propandiol, Ammoniak und Wasserstoff im molaren Verhältnis 1 : 60 : 2 wurde von oben nach unten durch den Reaktor gepumpt und bei einer Temperatur von 160 °C, einem Druck von 135 bar und einer Kontaktzeit (Definition siehe oben) von 40 000 g·s/mol zu 35% umgesetzt. Das Produkt enthielt 1,3-Diaminopropan mit einer Selektivität von 33% und 3-Amino-1-propanol mit einer Selektivität von 67%.

### Beispiel 3

### Herstellung von 1,3-Propandiamin

Es wurde verfahren wie in Beispiel 2 beschrieben, jedoch betrug die Reaktionstemperatur 195 °C und die Kontaktzeit 60 000 g·s/mol. der Umsatz war 95%, die Ausbeute an 1,3-Propandiamin 32%. Weiterhin wurden 8% 3-Amino-1-propanol und 40% sonstige Produkte gefunden.

### Vergleichsbeispiele 4 und 5

Das Reaktorsystem aus Beispiel 2 wurde mit 8 g eines kommerziellen Nickel-Trägerkatalysators (Engelhard Ni-6458) beschickt. Der Metallanteil dieses Katalysators beträgt 56%, der Träger besteht aus Siliciumdioxid. Dieser Katalysator besitzt nur einen geringen aciden Charakter, was durch Ammoniakadsorptionsmessungen bestimmt werden konnte. Ein Reaktionsgemisch aus 2,2-Dimethyl-1,3-propandiol, Ammoniak und Wasserstoff im molaren Verhältnis 1 : 60 : 2 wurde bei einer Temperatur von 210 °C, einem Druck von 135 bar und einer Kontaktzeit von 40 000 g·s/mol zu 84% umgesetzt. 1,3-Diamino-2,2-dimethylpropan wurde mit einer Selektivität von 70% erhalten, als Neben- bzw. Zwischenprodukt entstand 3-Amino-2,2-dimethyl-1-propanol mit einer Selektivität von 10%. Das Vergleichsbeispiel 5 wurde unter den gleichen Reaktionsbedingungen durchgeführt, ausser dass der Druck von 135 bar unter den kritischen Druck von Ammoniak (113 bar) auf 90 bar gesenkt wurde. Tabelle 1 zeigt die erhaltenen Resultate.

**Tabelle 1**

| Vergleichsbeispiele | Druck [bar] | Umsatz [%] | Selektivität [%] | | | |
|---|---|---|---|---|---|---|
| | | | Diamin | Aminol | Fragmente *) | Sonstige |
| 4 | 135 | 75 | 70 | 10 | 9 | 11 |
| 5 | 90 | 69 | 18 | 3 | 20 | 59 |

| | | | | | | |
|---|---|---|---|---|---|---|
| *) im flüssigen Produkt | | | | | | |

Tabelle 1 verdeutlicht den wesentlichen Einfluss des überkritischen Zustands auf die Selektivität der Aminierung. Sinkt der Druck von 135 bar auf 90 bar, so fällt die Selektivität der Diaminbildung von 70% auf 18%, während die Bildung von Nebenprodukten stark zunimmt.

### Vergleichsbeispiel 6

Analog zu Vergleichsbeispiel 4 wurde ein Reaktionsgemisch aus 1,1,1-Tris(hydroxymethyl)ethan, Ammoniak und Wasserstoff in einem molaren Verhältnis von 1 : 90 : 3 bei einer Temperatur von 195 °C, einem Druck von 135 bar und einer Kontaktzeit von 40 000 g·s/mol umgesetzt. Der Umsatz betrug 97%, die Ausbeute an 1,1,1-Tris(aminomethyl)ethan 13%.

### Vergleichsbeispiel 7

Das Reaktorsystem aus Beispiel 2 wurde mit 8 g eines kommerziellen Cobalt-Trägerkatalysators (Engelhard Co-0138) versehen. Der Metallanteil dieses Katalysators beträgt 25%, der Träger besteht aus Siliciumdioxid. Ein Reaktionsgemisch, bestehend aus 1,1,1-Tris-(hydroxymethyl)ethan, Ammoniak und Wasserstoff im molaren Verhältnis 1 : 90 : 3 wurde bei einer Temperatur von 185 °C, einem Druck von 135 bar und einer Kontaktzeit von 30 000 g·s/mol umgesetzt. Der Umsatz betrug 97%, die Ausbeute an 1,1,1-Tris(aminomethyl)ethan 10%.

## Patentansprüche

1. Verfahren zur Herstellung von primären aliphatischen Polyaminen der allgemeinen Formel 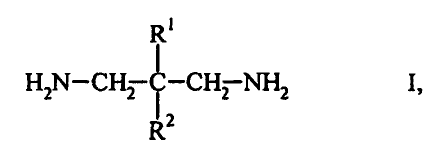 worin R¹ und R² unabhängig voneinander Wasserstoff, Methyl, Ethyl oder Aminomethyl bedeuten,
durch Umsetzung der entsprechenden Polyalkohole der allgemeinen Formel 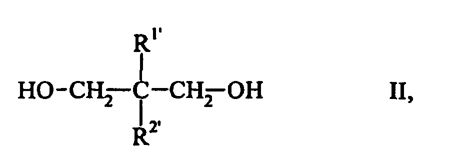 worin
R^{1'} = R¹ ist oder, falls R¹ Aminomethyl ist, R^{1'} auch Hydroxymethyl bedeuten kann, und R^{2'} = R² ist oder, falls R² Aminomethyl ist, R²' auch Hydroxymethyl bedeuten kann,
mit Ammoniak in Gegenwart von Wasserstoff, **dadurch gekennzeichnet, dass** die Umsetzung mit überkritischem Ammoniak in Gegenwart eines in seinem Metallanteil 5 bis 95 Gewichtsprozent Nickel und/oder Cobalt enthaltenden Katalysators, der zusätzlich einen Gehalt von 1 bis 60 Gewichtsprozent Fe und/oder La besitzt, bei einem Verhältnis von 1 bis 300 mol Ammoniak und 0,01 bis 20 mol Wasserstoff auf 1 val Alkohol durchgeführt wird.

2. Verfahren nach Anspruch 1, **dadurch gekennzeichnet, dass** der Katalysator durch Fällung der entsprechenden Hydroxide, Oxidhydrate und/oder basischen Carbonate bei einem pH von 5 bis 9 und anschliessende Trocknung und Kalzination bei 200 bis 500 °C in oxidierender Atmosphäre hergestellt wurde.

3. Verfahren nach Anspruch 1 oder 2, **dadurch gekennzeichnet, dass** der Katalysator auf einem Träger aus Siliciumdioxid, Kieselgur, Aluminiumoxid oder Graphit aufgebracht ist.

4. Verfahren nach einem der Ansprüche 1 bis 3, **dadurch gekennzeichnet, dass** der Katalysator bei einer Temperatur von 200 bis 400 °C mit Wasserstoff aktiviert wurde.

5. Verfahren nach einem der Ansprüche 1 bis 4, **dadurch gekennzeichnet, dass** der aliphatische Polyalkohol II ausgewählt ist aus der Gruppe bestehend aus 1,3-Propandiol, 2-Methyl-1,3-propandiol, 2,2-Dimethyl-1,3-propandiol, 1,1,1-Tris(hydroxymethyl)-ethan und Pentaerythrit.

6. Verfahren nach einem der Ansprüche 1 bis 5, **dadurch gekennzeichnet, dass** das Verfahren kontinuierlich durchgeführt wird.

7. Verfahren nach Anspruch 6, **dadurch gekennzeichnet, dass** die Kontaktzeit 10 000 bis 100 000 g·s/mol beträgt.

8. Verfahren nach Anspruch 6 oder 7, **dadurch gekennzeichnet, dass** der nicht umgesetzte Polyalkohol II und/oder das nicht umgesetzte Ammoniak rezykliert wird.

## Claims

1. Process for preparing primary aliphatic polyamines of the general formula 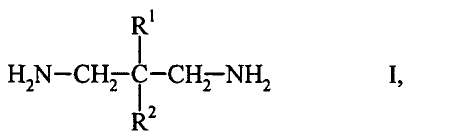 wherein R¹ and R² independently represent hydrogen, methyl, ethyl or aminomethyl,
by reacting the corresponding polyalcohols of the general formula 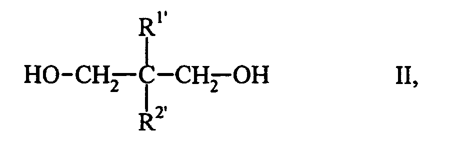 wherein R¹' = R¹ or, if R¹ is aminomethyl, R¹' may also be hydroxymethyl, and R^{2'} = R² or, if R² is aminomethyl, R²' may also be hydroxymethyl,
with ammonia in the presence of hydrogen, **characterized in that** the reaction is carried out with supercritical ammonia in the presence of a catalyst that contains 5 to 95 percent by weight of nickel/and or cobalt in its metallic component and, in addition, has a content of 1 to 60 percent by weight of Fe and/or La, at a ratio of from 1 to 300 mol of ammonia and from 0.01 to 20 mol of hydrogen per 1 val of alcohol.

2. Process according to Claim 1, **characterized in that** the catalyst was prepared by precipitation of the corresponding hydroxides, oxyhydrates and/or basic carbonates at a pH of 5 to 9 and subsequent drying and calcination at 200 to 500 °C in an oxidizing atmosphere.

3. Process according to Claim 1 or 2, **characterized in that** the catalyst is supported on a carrier consisting of silica, kieselguhr, alumina or graphite.

4. Process according to one of Claims 1 to 3, **characterized in that** the catalyst was activated with hydrogen at a temperature of 200 to 400 °C.

5. Process according to one of Claims 1 to 4, **characterized in that** the aliphatic polyalcohol II is selected from the group consisting of 1,3-propanediol, 2-methyl-1,3-propanediol, 2,2-dimethyl-1,3-propanediol, 1,1,1-tris(hydroxymethyl)ethane and pentaerythritol.

6. Process according to one of Claims 1 to 5, **characterized in that** it is carried out continuously.

7. Process according to Claim 6, **characterized in that** the contact time is from 10,000 to 100,000 g·s/mol.

8. Process according to Claim 6 or 7, **characterized in that** the unreacted polyalcohol II and/or the unreacted ammonia is recycled.

## Revendications

1. Procédé pour la préparation de polyamines primaires aliphatiques de formule générale 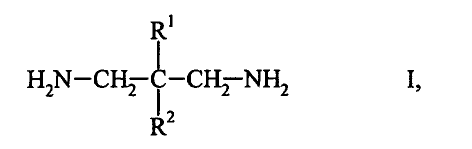 dans laquelle R¹ et R² représentent indépendamment l'hydrogène, le méthyle, l'éthyle ou l'aminométhyle,
par la réaction de les polyalcools correspondants de formule générale 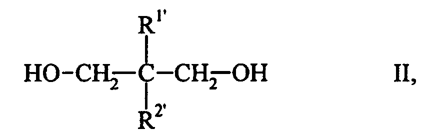 dans laquelle R¹' est égal à R¹ ou, si R¹ est aminométhyle, R^{1'} peut aussi signifier hydroxyméthyle, et R²' est égal à R² ou, si R² est aminométhyle, R²' peut aussi signifier hydroxyméthyle, avec l'ammoniac en présence d'hydrogène, **caractérisé en ce que** la réaction se fait avec l'ammoniac supercritique en présence d'un catalyseur, qui dans sa fraction métallique contient de 5 à 95 pour cent de poids en nickel et/ou cobalt, et comprend de 1 à 60 pour cent de poids additionnel en Fe et/ou La, pour un rapport de 1 à 300 mol d'ammoniac et de 0,01 à 20 mol d'hydrogène par 1 val d'alcool.

2. Procédé selon la revendication 1, **caractérisé en ce que** le catalyseur a été préparé par précipitation des hydroxydes, oxyhydrates et/ou carbonates basiques correspondants à un pH de 5 à 9 et séchage subséquent ainsi que calcination en atmosphère oxydante à 200-500 °C.

3. Procédé selon la revendication 1 ou 2, **caractérisé en ce que** le catalyseur est appliqué sur un support en dioxyde de silicium, kieselguhr, oxyde d'aluminium ou graphite.

4. Procédé selon l'une des revendications 1 à 3, **caractérisé en ce que** le catalyseur a été activé avec hydrogène à une température de 200 à 400 °C.

5. Procédé selon l'une des revendications 1 à 4, **caractérisé en ce que** le polyalcool aliphatique II est choisi dans le groupe constitué de propanediol-1,3, méthyl-2 propanediol-1,3, diméthyl-2,2 propanediol-1,3, tris(hydroxyméthyl)-1,1,1 éthane et pentaérythrite.

6. Procédé selon l'une des revendications 1 à 5, **caractérisé en ce que** qu'il est mis en oeuvre en continu.

7. Procédé selon l'une des revendications 1 à 6, **caractérisé en ce que** le temps de contact va de 10 000 à 100 000 g·s/mol.

8. Procédé selon la revendication 6 ou 7, **caractérisé en ce que** le polyalcool II n'ayant pas réagi et/ou l'ammoniac n'ayant pas réagi est(sont) recyclé(s).
